Europäisches Patentamt

(19) **European Patent Office**

Office européen des brevets

(11) Publication number: **0 019 371**

**B1**

(12) **EUROPEAN PATENT SPECIFICATION**

(45) Date of publication of patent specification: **01.02.84**

(51) Int. Cl.³: **A 61 L 15/00**

(21) Application number: **80301307.7**

(22) Date of filing: **23.04.80**

(54) **Absorbent materials, process for making them and their use in immobilising blood.**

(30) Priority: **23.04.79 GB 7914067**

(43) Date of publication of application:
**26.11.80 Bulletin 80/24**

(45) Publication of the grant of the patent:
**01.02.84 Bulletin 84/5**

(84) Designated Contracting States:
**AT BE DE FR GB NL**

(56) References cited:
**DE - A - 2 702 781**
**DE - A - 2 737 994**
**FR - A - 1 293 415**
**FR - A - 2 302 752**
**FR - A - 2 305 452**
**FR - A - 2 310 156**
**FR - A - 2 331 592**
**LU - A - 68 382**

(73) Proprietor: **UNILEVER PLC**
**Unilever House Blackfriars  P O Box 68**
**London EC4P 4BQ (GB)**
(84) **GB**
(73) Proprietor: **UNILEVER NV**
**Burgemeester s'Jacobplein 1 P.O. Box 760**
**NL-3000 DK  Rotterdam (NL)**
(84) **BE DE FR NL AT**

(72) Inventor: **Windust, John Herbert Charles**
**79 Curlew Crescent**
**Brickhill Bedford (GB)**

(74) Representative: **Doucy, Robert Henry et al,**
**Unilever PLC Patents Division P.O. Box 68**
**Unilever House**
**London EC4P 4BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Absorbent materials, process for making them and their use in immobilising blood

This invention relates to absorbent materials and more particularly to absorbent materials for inclusion in products intended for the absorption of blood (which is to be understood herein as including menstrual fluid), such as sanitary towels and tampons, and in various other disposable absorbent products including those for medical and surgical usage. The invention also relates to processes for making the absorbent materials and to their use for immobilising blood.

In recent years a number of synthetic and partly synthetic water-swellable, substantially water-insoluble, cross-linked polyelectrolytes have been proposed for use in disposable products because of their ability to absorb substantial amounts of body fluids, particularly water, urine and blood. These absorbent materials are able to bind body fluids irreversibly so that absorbed fluid is rendered immobile and cannot be squeezed out under the pressures normally associated with the use of absorbent disposable products. While many such highly absorbent products can be used satisfactorily for the immobilisation of water, they are much less effective for immobilising urine and blood. In addition, while the absorbents can absorb the aqueous phase of blood and the lower molecular weight components thereof, blood also contains a high concentration of high molecular weight proteins which are not capable of being absorbed by these absorbent materials. Consequently, in a product containing the absorbent material, some of the blood tends to remain at the outside of a mass of the absorbent particles and in a fluid or mobile state. This fluid blood material can therefore leak from an absorbent product or cause staining, both of which are disadvantageous.

It is an object of the invention to provide an absorbent material particularly suitable for immobilising blood.

According to the invention there is provided a blood swellable, substantially water-insoluble, covalently cross-linked anionic polyelectrolyte having at least some of its anions associated with transition metal counterions which are capable of coagulating blood, the polyelectrolyte being characterised by the property that when the polyelectrolyte is contacted with blood transition metal counterions dissociate from the polyelectrolyte, diffuse into the blood and cause coagulation thereof. This coagulation, which we believe to be due to the cross-linking of blood proteins by the transition metal counterions, is to be distinguished from the well known clotting process which is the normal response to wounding and which is initiated by calcium ions.

The anionic polyelectrolyte is preferably an anionic polysaccharide, for example an anionic derivative of starch, dextran or cellulose. While the transition metal ions will effect ionic cross-linking of anionic groups, the polyelectrolyte is covalently cross-linked whereby even in the absence of the transition metal ions the anionic polyelectrolyte is at least partially water-insoluble, eg at least 40% by weight water-insoluble. In a preferred form, the unmodified absorbent has a water-insolubility of at least 90% by weight. The covalent cross-linking may be effected by internal esterification as in US Patent Specification No. 3 678 931 (Buckeye Cellulose Corporation) or by suitable cross-linking agents which are well known to those in the art and include those referred to in German Patent Application No. P 2 702 781.2 (Unilever), German Patent Application No. P 2 634 539.1 (Hoechst AG), British Patent Specification No. 936 039 (Pharmacia) and US Patent Specification No. 3 589 364 (Buckeye Cellulose Corporation). Such cross-linking agents include the bifunctional compounds Q-R-Y where R is an alkylene group containing 1 to 10, and especially 2 to 5, carbon atoms, which may be substituted by one or more hydroxy groups, and/or which may be interrupted by heteroatoms, eg oxygen atoms, and Q and Y each represent a halogen atom or an epoxy oxygen atom, the latter being linked to carbon atoms of the group R with the formation of an oxirane ring. Examples of such agents are epichlorhydrin, dichlorohydrin, dibromohydrin, 1,2-3,4-diepoxybutane, 1,2- 7,8-diepoxyoctane, bisepoxypropylether and 1,4-butane-diol-bisepoxypropylether.

The anionic groups are preferably carboxyalkyl groups, especially carboxymethyl or carboxyethyl groups. Polysaccharide compounds preferably have a degree of substitution of anionic groups of at least 0.1, more preferably at least 0.2.

In the absorbent material of this invention at least some of the anions of the anionic polyelectrolyte are associated, or ionically complexed, with a transition metal counterion having special properties. Transition elements include elements 21 to 30 (scandium to zinc), 39 to 48 (yttrium to cadmium) and 57 to 80 (lanthanum to mercury) and the transition metal counterion present in the absorbent material of this invention is one which has the property of being able to cause the coagulation of blood. Transition metal ions effective for this purpose are known in the art and include especially copper and zinc ions although others such as nickel ions have this property to some extent. The bringing together of these coagulating ions and blood when using the absorbent material of this invention occurs simultaneously with the removal of part of the aqueous absorbable fraction of blood by the absorbent. By this means the immobilising action of the transition metal ions on the unabsorbed blood proteins by the coagulant effect is enhanced. Consequently, the absorbent material of the invention immobilises blood by two distinct mechanisms: 1) the absorption by the absorbent of an aqueous fraction containing low molecular weight components; and 2) the gelation of a high molecular weight protein fraction which is not absorbed. A further factor contributing to the effectiveness of the absorbent material of this invention in the immobilisation of unabsorbed blood is the binding action by the gel formed by the association of the blood proteins with the transition metal ions on the residual aqueous fluids.

It is also to be appreciated that it is an essential feature of the absorbent material of the present invention that the blood-coagulating transition metal ions are present in such manner that when the absorbent material is contacted by blood they can diffuse from the polymer and cause gelation of blood proteins. Therefore the anionic groups of the polyelectrolyte must not bind too tightly the transition metal ions and the polymer network should not prevent the diffusion of the ions from the absorbent material to effect the desired coagulation of blood. The coagulant effect of the transition metal ions diffusing into blood is readily observable in the microscope as is more particularly described hereinafter.

Absorbent materials in accordance with this invention will generally contain at least 0.5 milliequivalents (meq) of the blood coagulating metal per gram, for example 0.5 to 10 meq/g.

It is not essential that all the anions of the polyelectrolyte be associated with the transition metal ions having a blood coagulating effect. Some of the anions may be associated with other counterions, for example alkali metal, alkaline-earth metal, ammonium or substituted ammonium ions, for example sodium, potassium, calcium, ammonium and tetramethylammonium ions.

Preferred absorbent materials of this invention are the zinc and copper salts of water-swellable, covalently cross-linked carboxymethyl starch, dextran or cellulose.

Of special usefulness are the zinc salt forms of the absorbent materials described in German Patent Application No. P 2,702,781.2. However, also of particular usefulness are the zinc salt forms of the cross-linked carboxymethyl starch-, dextran- and cellulose-based water-swellable absorbent materials described in the Hoechst, Pharmacia and Buckeye Cellulose Corporation patent specifications referred to above.

The absorbent product of the invention is preferably in particulate form such as in the form of a powder, granules, flakes or fibres.

The invention also relates to processes for making the absorbent materials of this invention.

Accordingly the invention also relates to a process for making an absorbent material of the invention in which process a water-swellable, at least partially water-insoluble, covalently cross-linked anionic polyelectrolyte is treated in a water swollen state with a compound of a transition metal whose ions are capable of coagulating blood so that at least some of the anions of the polyelectrolyte become associated with transition metal counterions, and the treated polyelectrolyte is then dried.

According to one such process a water-swellable, covalently cross-linked, at least partially water-insoluble, anionic polyelectrolyte in its acid form is contacted in a water-swollen state with a compound of said transition metal reactive with the acidic groups of said polyelectrolyte to form metal salt groups; any non-volatile anions of the metal compound are removed by washing, and the water-swollen absorbent then dried. In this process the transition metal compound may be water-soluble or water-insoluble. Examples of suitable compounds are the basic zinc and copper compounds such as the carbonates, oxides and hydroxides of these metals.

The acid form of the polyelectrolyte may be obtained by the acid washing of a salt form.

In an alternative process a water-swellable, covalently cross-linked, at least partially water-insoluble, anionic polyelectrolyte in the form of its alkali metal, alkaline-earth metal, ammonium or substituted ammonium salt is contacted with a medium in which the polyelectrolyte is swellable and in which a soluble salt of said transition metal is dissolved whereby ion-exchange occurs and ions of said transition metal are introduced into the swollen polyelectrolyte, whereafter the polyelectrolyte is separated from said medium, washed and then dried. The swelling medium for the polyelectrolyte is preferably water but suitable aqueous alcoholic media if they contain sufficient water to permit the polyelectrolyte to swell can also be employed; examples of alcohols for such media are methanol, ethanol and propanol. Examples of suitable transition metal salts for use in this process are the chlorides, nitrates, sulphates and acetates of copper and zinc although any other substantially water-soluble salt of the transition metal can be used.

The introduction of the transition metal ions into the absorbent material in the above processes will, where they replace monovalent cations, act to further cross-link temporarily the polyelectrolyte and this will enhance the wet-out of a mass of the absorbent by blood. However, if desired, the absorbent may also be surface-treated to improve blood wet-out with a polyether as described in British Patent Application Serial No. 2 007 998 or with one of the hydrocarbon materials described in European Patent Publication No. 9977.

The invention also relates to the use in absorbent articles for immobilising blood of the absorbent material of the invention. The absorbent article may comprise a fibrous carrier or support for the absorbent material, such as a woven or unwoven material such as cotton cloth, rayon, wool, surgical gauze or paper as well as cellulosic fluff, on or within which the absorbent material is supported. The absorbent material may be spread on the carrier or it may be mixed with loose fibres to make a composite fluff or wadding which can be enclosed between cover sheets of paper or cloth. The article may also be in the form of a laminate. In a particular form, the carrier comprises two sheets between which the absorbent material is sandwiched.

The Applicants are aware that it has already been proposed in US Patent Specification No. 4 043 952 (National Starch and Chemical Corporation) to surface-treat a particulate water-swellable anionic polyelectrolyte in a medium in which the polyelectrolyte does not swell with a zinc salt to ionically complex the particle surface by zinc ions. The water-swellable, anionic polyelectrolytes surface-treated in said US Patent Specification No. 4 043 052 are of three kinds, viz water-soluble materials; water-insoluble, covalently cross-linked materials; and water-insoluble, ionically complexed materials. As described hereinafter, we have surface-treated various absorbents in accordance with the procedure described in said patent specification with a zinc compound but the treated absorbents have not displayed the critical property characteristic of the absorbent materials of this invention, that is that the blood-coagulating ion dissociates from the polyelectrolyte, diffuses into blood and causes coagulation thereof.

The invention will now be illustrated by the following Examples of which Examples 1 to 10 describe the preparation of absorbents according to the invention whereas Examples A to E concern absorbents not according to this invention but which are included for comparative purposes. Percentages are by weight unless otherwise specified.

Example 1
Potato starch (1000 g) was slurried in water (950 ml) containing epichlorohydrin (8.4 ml; 1.0% epichlorohydrin by weight of starch). Sodium hydroxide (5 g) in water (50 ml) was added with stirring and the mixture was applied to a heated roller via a feeder roller to form a layer on the surface of the roller of about 0.5 mm thickness. The roller itself was heated using steam at 40 lbs/sq. in gauge (3.77 bars) (140°C). The cross-linked starch derivative was removed from the roller as a flake material.

Sodium hydroxide (34 g) in water (66 ml) followed by monochloracetic acid (39 g) in water (11 ml) was slowly added with stirring to the cross-linked potato starch (100 g) as prepared above. The mixture was aged overnight in a polythene bag. The theoretical degree of substitution was 0.67.

The moist carboxymethyl derivative was dispersed in 10 times its weight of $1N$ hydrochloric acid and soaked for 15 min. then filtered. The gel cake was repeatedly dispersed in water and filtered until the filtrate was substantially free of chloride ions. Ammonium hydroxide, specific gravity 0.910, (70 ml) was mixed with the water swollen washed acid cake before drying in a forced air oven (70°C).

The absorbent (50 g) was soaked in an aqueous solution of zinc nitrate ($2\frac{1}{2}$ l, $1M$) overnight, filtered, washed until the filtrate was substantially free of nitrate ions then dried in a forced air oven at 70°C and milled through a 2 mm screen.

Example 2
Example 1 was repeated as far as ageing the carboxymethylated mixture in a polythene bag.

The moist carboxymethyl derivative was repeatedly dispersed in water and filtered until the filtrate was neutral. The gel cake was then dispersed in an aqueous solution of copper sulphate 5 l, $1M$), soaked overnight, the washed and filtered until the filtate was substantially free of sulphate ions, and dried in a forced air oven at 70°C.

Example 3
Example 1 was repeated except that after washing of the acid cake, basic zinc carbonate (10.8 g, 100% neutralisation) was thoroughly stirred into the acid cake and the product dried in a forced air oven at 70°C.

Example 4
Example 3 was repeated but zinc oxide (7.75 g, 100% neutralisation) was stirred into the acid cake before drying.

Example 5
Example 3 was repeated except enough basic zinc carbonate was added to the cake to give theoretically 80% neutralisation of the acid groups, the remainder being neutralized by stirring in the appropriate quantity of ammonia solution (specific gravity 0.910).

Example 6
Example 1 was repeated but the absorbent (100 g) was soaked in nickel chloride solution (5 l, $1M$) overnight, filtered, washed until the filtrate was substantially free of chloride ions, then dried in a forced air oven at 70°C.

Examples 7—10
Absorbant (10 g), as detailed below, was soaked in copper sulphate solution (500 ml, $1M$) overnight, washed until the filtrate was substantially free of sulphate ions, dried in a forced air oven at 70°C and then milled through a 2 mm screen. The following absorbents were used in these Examples:

| Example | Absorbent |
|---------|-----------|
| 7 | P |
| 8 | Q |
| 9 | R |
| 10 | S |

Absorbent P was a carboxymethylated cellulose cross-linked by intermolecular esterification and available from Hercules Corporation under the trade name "Aqualon R" and generally described by Podlas, T. J. in INDA Tech. Symp. 1976, 2—3 March, pp 25—39.

Absorbent Q was a cross-linked sodium carboxymethyl cellulose obtained from Courtaulds Limited under the name of "Superabsorbent Blend DP 1006".

Absorbent R was a cross-linked sodium carboxymethyl cellulose obtained from Hoechst AG under the designation "2608/011".

Absorbent S was a cross-linked carboxymethyl dextran obtained from Pharmacia under the name of "CM-Sephadex C-50".

Comparative Examples A to C

Absorbent (20 g), as detailed below, was added to a 500 cm³ conical flask equipped with stirrer, water bath and condenser. Methanol (75 ml) was added followed by sufficient zinc acetate to give a cation level of 10 milliequi-valents $Zn^{2+}$ per gram of absorbent on a dry weight basis. The mixture was stirred and heated to 68°C for 1 hour before cooling, filtering and drying.

Comparative Examples A and B illustrate the surface complexing of water-insoluble, covalently cross-linked materials and Comparative Example C illustrates the surface complexing of a water-insoluble, ionically complexed material.

The following absorbents were used in these Examples:

| Comparative Example | Absorbent |
|---------------------|-----------|
| A | T |
| B | U |
| C | V |

Absorbent T is the material as prepared in Example 1 but without the zinc treatment.

Absorbent U was a hydrolysed starch-polyacrylonitrile graft copolymer available from General Mills Inc., under the trade name "SGP-502S" and generally described in US Patent No. 3,997,484.

Absorbent V was the potassium salt of a polyacrylic acid cross-linked by aluminium ions available commercially from National Starch Corporation under the trade name "Permasorb 30" and generally described in US Patent No. 4,090,013.

Comparative Example D

Example 1 was repeated as far as ageing the carboxymethylated mixture in a polythene bag. The moist carboxymethyl derivative was repeatedly dispersed in water and filtered until the filtrate was neutral. The gel cake was then dried in a forced air oven at 70°C and milled through a 2 mm screen.

Comparative Example E

Example 1 was repeated but instead of being treated with zinc nitrate the absorbent (100 g) was soaked in aluminium sulphate solution (5 l, 0.5 M) overnight, washed and filtered until the filtrate was substantially free of sulphate ions, dried in a forced air oven at 70°C and milled through a 2 mm screen.

All of the absorbents referred to in the above examples were, prior to treatment with the transition metal compound, insoluble in water to the extent of at least 50% by weight. The treated materials were highly water-insoluble.

The blood coagulating property of the absorbents of Examples 1 to 10 and especially the contrast therewith of the effect of the absorbents of the Comparative Examples A to E was readily observed under the microscope. The examinations were made using a Leitz Ortholux II microscope under "dark field" or "phase contrast" conditions and a magnification of 250 times. The test fluid used was bovine serum albumin (5%) in sodium chloride solution (0.9%) rather than haemolysed blood because of its more constant composition, greater stability to microbiological attack and its optical transparency.

One or two drops of the bovine serum albumin test fluid were placed on a microscope slide and then a small quantity of the absorbent material placed on top, the whole being covered with a cover glass. The absorbent material was then studied for up to 1 hour.

Taking the results of all the examinations as a whole three types of effect could be clearly distinguished.

Effect 1—There immediately begins to form an area of gelling protein adjacent to the particles, which area becomes both larger and denser with time.

Effect 2—A slight precipitate was seen after about 30 minutes as a brightening of the surface of the particles, this probably being due to absorbed protein.

Effect 3—No precipitate observed.

Effect 1 is displayed by absorbents according to the invention.

Similar effects are obtained using serum prepared from blood.

The following Table 1 lists the effects produced by the materials of the various examples and indicates the nature of the counterion of the respective polyelectrolyte absorbent.

### TABLE 1

| Example | Counterion | Type of effect |
|---|---|---|
| 1 | $Zn^{2+}$ | 1 |
| 2 | $Cu^{2+}$ | 1 |
| 3 | $Zn^{2+}$ | 1 |
| 4 | $Zn^{2+}$ | 1 |
| 5 | $Zn^{2+}/NH_4^+$ | 1 |
| 6 | $Ni^{2+}$ | 1 |
| 7 | $Cu^{2+}$ | 1 |
| 8 | $Cu^{2+}$ | 1 |
| 9 | $Cu^{2+}$ | 1 |
| 10 | $Cu^{2+}$ | 1 |
| A | $NH_4^+$ $(Zn^{2+})$* | 2 |
| B | $Na^+$ $(Zn^{2+})$* | 2 |
| C | $K^+/Al^{3+}$ $(Zn^{2+})$* | 2 |
| D | $Na^+$ | 3 |
| E | $Al^{3+}$ | 3 |

*$(Zn^{2+})$ indicates zinc ion at particle surface

Urine retention values and blood retention values for the materials of the above examples were determined by the following methods.

**Determination of urine retention value**

The sample to be tested (0.20 g) is weighed out into a pre-weighed sintered glass Gooch crucible. Synthetic urine (5 ml) is added to the sample ensuring that the sample is completely wetted and it is left to soak for the prescribed time before being placed in a centrifuge tube and spun for 10 minutes at 850 rpm in a centrifuge having a head radius of 9 cm. The crucible with contents is then reweighed. The urine retention value is expressed as the weight of urine retained per gram of dry absorbent. The formula of synthetic urine, derived from the information given in the Handbook of Clinical Laboratory Data, 2nd Edition, 1968, pages 17—20, is a solution of 5 litres of water of the following:

| | Grams |
|---|---|
| $CaCl_2 . 2H_2O$ | 3.680 |
| $K_2SO_4$ | 0.175 |
| KCl | 44.740 |
| KOH | 2.190 |
| $NH_4Cl$ | 6.020 |
| Citric acid | 2.630 |

Experiments have shown that the same urine retention values are obtained by using natural urine in place of the synthetic urine.

**Determination of blood retention value**

To determine the blood retention value, the sample to be tested (0.20 g) is weighed out and sprinkled onto haemolysed blood (5—10 ml) in a 25 ml glass beaker. After leaving to soak for the prescribed time, the contents are transferred to a pre-weighed sintered glass Gooch crucible and placed in the centrifuge tube and spun in the same manner as described above. The crucible with contents is then reweighed. The blood retention value is expressed as the weight of blood retained per gram of dry absorbent.

Usually the blood and urine retention values of absorbent materials are very similar or equivalent but in the case of the absorbent materials of the invention the blood retention value rises with time and reaches a maximum within about 2 hours. The maximum is occasionally reached in a considerably shorter time and hence the blood retention value measured after 10 minutes can be similar to that measured after 2 hours. Similar results are obtained using menstrual fluid in place of blood.

These retention measurements effectively measure the amount of material (urine or blood) rendered immobile under the conditions of the test. Consequently, the blood retention value will take account of blood which while not absorbed within the particles of the poly-electrolytic absorbent is coagulated and rendered immobile even under those conditions of the test which are designed to remove absorbate between the particles of the absorbent. The higher blood retention values after the contact period of 2 hours for the absorbent materials of this invention are indicative of the increase in the degree of immobilisation of blood obtained with these materials.

During the determination of the blood retention value the swelling of the absorbent material may tend to block the pores in the sinter glass crucible and hinder non-bound fluid from draining during centrifugation. For this reason, the qualitative microscopic examination is a better way of determining or demonstrating a coagulating effect on blood of an absorbent containing metal ions. The microscopic examination method must also be relied upon to differentiate absorbents of the invention from those absorbents, such as Absorbent V, which are ionically cross-linked by polyvalent ions who do not have the property of coagulating blood, since these absorbents also have a swelling capacity on exposure to blood which increases with time (and therefore the blood retention value increases in time) due to diffusion of cross-linking ions from the absorbent particles.

The determined urine retention value (URV) and blood retention values (BRV) for the products of the above examples are given in Table 2 which also includes the number of milliequivalents of cation per gram of dry polymer. The retention values are given to the nearest whole number.

TABLE 2

| Example | URV (2 hr soak) | BRV (10 min soak) | BRV (2 hr soak) | meq cation | Cation |
|---|---|---|---|---|---|
| 1 | 7 | 13 | 22 | 2.21 | Zn |
| 2 | 2 | 21 | 32 | 2.79 | Cu |
| 3 | 7 | 11 | 22 | 1.77 | Zn |
| 4 | 6 | 13 | 31 | 2.11 | Zn |
|  |  |  |  | 2.09 | Zn |
| 5 | 7 | 13 | 36 | 0.33 | NH$_4$ |
| 6 | 5 | 7 | 11 | 2.26 | Ni |
| 7 | 3 | 10 | 16 | 2.37 | Cu |
| 8 | 1 | 6 | 9 | 4.86 | Cu |
| 9 | 2 | * | 23 | 3.02 | Cu |
| 10 | 2 | 4 | 29 | 3.49 | Cu |
|  |  |  |  | 0.26 | NH$_4$ |
| A | 9 | 10 | 10 | 0.16 | Zn |
|  |  |  |  | 2.69 | Na |
| B | * | * | 24 | 1.95 | Zn |
|  |  |  |  | 5.41 | K |
| C | 14 | 15 | 29 | 0.17 | Zn |
|  |  |  |  | 0.25 | Al |
| D | 12 | 12 | 12 | 2.29 | Na |
| E | 2 | 1 | 4 | 0.32 | Al |

*value unobtainable due to poor drainage of the swollen material in the centrifugation.

## Claims

1. An absorbent material suitable for immobilising blood consisting of a blood-swellable, sustantially water-insoluble, covalently cross-linked anionic polyelectrolyte having at least some of its anions associated with transition metal counterions which are capable of coagulating blood, the polyelectrolyte being characterised by the property that when the polyelectrolyte is contacted with blood transition metal counterions dissociate from the polyelectrolyte, diffuse into the blood and cause coagulation thereof.

2. An absorbent material as claimed in Claim 1, characterised in that the polyelectrolyte is an anionic polysaccharide.

3. An absorbent material as claimed in Claim 1 or Claim 2, characterised in that the anionic polyelectrolyte has anionic groups which are carboxyalkyl groups.

4. An absorbent material as claimed in any of Claims 1 to 3, characterised in that the transition metal is zinc or copper.

5. Process for making an absorbent material as claimed in Claim 1, characterised in that a water-swellable, at least partially water-insoluble, covalently cross-linked anionic polyelectrolyte is treated in a water-swollen state with a compound of a transition metal whose ions are capable of coagulating blood so that at least some of the anions of the polyelectrolyte become associated with transition metal counterions, and the treated polyelectrolyte is then dried.

6. A process as claimed in Claim 5, characterised in that the anionic polyelectrolyte in its acid form is contacted in a water-swollen state with a transition metal compound reactive with the acidic groups of said polyelectrolyte to form metal salt groups; any non-volatile anions of the metal compound are removed by washing, and the water-swollen absorbent then dried.

7. A process as claimed in Claim 6, characterised in that the transition metal compound is a basic zinc or copper compound.

8. A process as claimed in Claim 7, characterised in that the transition metal compound is zinc or copper carbonate, oxide or hydroxide.

9. A process as claimed in Claim 5, characterised in that the anionic polyelectrolyte in the form of its alkali metal, alkaline-earth metal, ammonium or substituted ammonium salt is contacted with a medium in which the polyelectrolyte is swellable and in which a soluble salt of said transition metal is dissolved whereby ion-exchange occurs and ions of said transition metal are introduced into the swollen polyelectrolyte, whereafter the polyelectrolyte is separated from said medium, washed and then dried.

10. A process as claimed in Claim 9, characterised in that the swelling medium for the polyelectrolyte is water.

11. A process as claimed in Claim 9 or 10, characterised in that the transition metal salt is zinc or copper chloride, nitrate, sulphate or acetate.

12. Non-medical and non-surgical use of an absorbent material as claimed in Claim 1 for immobilising blood.

7

## Patentansprüche

1. Absorbierendes Material, geeignet zum Stillen von Blut, bestehend aus einem durch Blut quellbaren, im wesentlichen wasserunlöslichen, kovalent vernetzten anionischen Polyelektrolyten, von dem zumindest einige seiner Anionen mit Übergangsmetall-Gegenionen assoziiert sind, die Blut zu koagulieren vermögen, wobei sich der Polyelektrolyt durch die Eigenschaft auszeichnet, daß, wenn der Polyelektrolyt mit Blut in Berührung kommt, Übergangsmetall-Gegenionen von dem Polyelektrolyten dissoziieren, in das Blut diffundieren und dessen Koagulation verursachen.

2. Absorbierendes Material nach Anspruch 1, dadurch gekennzeichnet, daß der Polyelektrolyt ein anionisches Polysaccharid ist.

3. Absorbierendes Material nach Anspruch 1 oder Anspruch 2, dadurch gekennzeichnet, daß der anionische Polyelektrolyt anionische Gruppen aufweist, die Carboxyalkylgruppen sind.

4. Absorbierendes Material nach irgend einem der Ansprüche 1 bis 3, dadurch gekennzeichnet, daß das Übergangsmetall Zink oder Kupfer ist.

5. Verfahren zur Herstellung eines absorbierenden Materials nach Anspruch 1, dadurch gekennzeichnet, daß ein mit Wasser quellbarer, wenigstens teilweise wasserunlöslicher, kovalent vernetzter anionischer Polyelektrolyt in einem mit Wasser gequollenen Zustand mit einer Verbindung eines Übergangsmetalls behandelt wird, dessen Ionen Blut zu koagulieren vermögen, so daß wenigstens einige der Anionen des Polyelektrolyten mit Übergangsmetall-Gegenionen assoziieren, und der behandelte Polyelektrolyt dann getrocknet wird.

6. Verfahren nach Anspruch 5, dadurch gekennzeichnet, daß der anionische Polyelektrolyt in seiner sauren Form in einem mit Wasser gequollenen Zustand mit einer Übergangsmetallverbindung zusammengebracht wird, die mit den sauren Gruppen des Polyelektrolyten zu Metallsalzgruppen reagiert; irgendwelche nichtflüchtige Anionen der Metallverbindung durch Waschen entfernt werden und das mit Wasser gequollene Absorptionsmittel dann getrocknet wird.

7. Verfahren nach Anspruch 6, dadurch gekennzeichnet, daß die Übergangsmetallverbindung eine basische Zink- oder Kupferverbindung ist.

8. Verfahren nach Anspruch 7, dadurch gekennzeichnet, daß die Übergangsmetallverbindung Zink- oder Kupfercarbonat, -oxid oder -hydroxid ist.

9. Verfahren nach Anspruch 5, dadurch gekennzeichnet, daß der anionische Polyelektrolyt in Form seines Alkalimetall-, Erdalkalimetall-, Ammonium- oder subst. - Ammoniumsalzes mit einem Medium zusammengebracht wird, in dem der Polyelektrolyt quellbar ist und in dem ein lösliches Salz des Übergangsmetalls gelöst ist, wodurch Ionenaustausch eintritt und Ionen des Übergangsmetalls in den gequollenen Polyelektrolyten eingeführt werden, worauf der Polyelektrolyt von dem Medium abgetrennt, gewaschen und dann getrocknet wird.

10. Verfahren nach Anspruch 9, dadurch gekennzeichnet, daß das quellende Medium für den Polyelektrolyten Wasser ist.

11. Verfahren nach Anspruch 9 oder 10, dadurch gekennzeichnet, daß das Übergangsmetallsalz Zink- oder Kupferchlorid, -nitrat, -sulfat oder -acetate ist.

12. Nicht-medizinische und nicht-chirurgische Verwendung eines absorbierenden Materials gemäß Anspruch 1 zum Stillen von Blut.

## Revendications

1. Un matériau absorbant approprié pour une immobilisation du sang constitué d'un polyélectrolyte anionique, réticulé par covalence, pratiquement insoluble dans l'eau, gonflant dans le sang ayant au moins une partie de ses anions associée avec des ions complémentaires de métal de transition qui sont capables de coagular du sang, le polyélectrolyte étant caractérisé par la propriété que, quand le polyélectrolyte est mis en contact avec du sang, des ions complémentaires de métaux de transition se dissocient du polyélectrolyte, diffusent dans le sang et provoquent une coagulation de celui-ci.

2. Un matériau absorbant selon la revendication 1, caractérisé en ce que le polyélectrolyte est un polysaccharide anionique.

3. Un matériau absorbant selon la revendication 1 ou 2, caractérisé en ce que le polyélectrolyte anionique possède des groupes anioniques qui sont des groupes carboxyalkyle.

4. Un matériau absorbant selon l'une des revendications 1 à 3, caractérisé en ce que le métal de transition est du zinc ou du cuivre.

5. Procédé de fabrication d'un matériau absorbant selon la revendication 1, caractérisé en ce qu'un polyélectrolyte anionique, réticulé par covalence, au moins partiellement insoluble dans l'eau, gonflant dans l'eau est traité dans un état gonflé d'eau avec un composé d'un métal de transition dont les ions sont capables de coaguler du sang de sorte qu'au moins une partie des anions du polyélectrolyte devienne associée avec des ions complémentaires de métal de transition, et le polyélectrolyte traité est ensuite séché.

6. Un procédé selon la revendication 5, caractérisé en ce que le polyélectrolyte anionique dans sa forme acide est mis en contact dans un état gonflé d'eau avec un composé de métal de transition réactif avec les groupes acides du dit polyélectrolyte pour former des groupes de sel de métal; les anions non volatiles du composé métallique sont enlevés par lavage, et l'absorbant gonflé d'eau est ensuite séché.

7. Un procédé selon la revendication 6, caractérisé en ce que le composé de métal de transition est un composé de zinc ou de cuivre basique.

8. Un procédé selon la revendication 7, caractérisé en ce que le composé de métal de transition est du carbonate, de l'oxyde ou de l'hydroxyde de zinc ou de cuivre.

9. Un procédé selon la revendication 5, caractérisé en ce que le polyélectrolyte anionique sous la forme de son sel de métal alcalin, de métal alcalino-terreux, d'ammonium ou d'ammonium substitué est mis en contact avec un milieu dans lequel le polyélectrolyte peut gonfler et dans lequel un sel soluble du dit métal de transition set dissous, grâce à quoi un échange d'ion se produit et des ions du dit métal de transition sont introduits dans le polyélectrolyte gonflé, à la suite de quoi le polyélectrolyte est séparé du dit milieu, lavé et ensuite séché.

10. Un procédé selon la revendication 9, caractérisé en ce que le milieu permettant le gonflement du polyélectrolyte est de l'eau.

11. Un procédé selon les revendications 9 ou 10, caractérisé en ce que le sel de métal de transition est du chlorure, du nitrate, du sulfate ou de l'acétate de zinc ou de cuivre.

12. Utilisation non médicale et non chirurgicale d'un matériau absorbant selon la revendication 1 pour immobiliser du sang.